# EUROPEAN PATENT APPLICATION

(11) **EP 1 489 406 A1**
(43) Date of publication of application: **22.12.2004**
(21) Application number: 03101829.4
(22) Date of filing: 20.06.2003
(51) Int. Cl.: G01N 21/64, G01N 21/65, G01J 3/28, A61B 1/04

(54) **Spectroscopic imaging system**

(71) Applicant: EUROPEAN COMMUNITY, 1049 Brussels (BE)
(72) Inventor: Whelan, Maurice, 21021, ANGERA (IT); Bouhifd, Mounir, 67100 Strasbourg (FR)
(74) Representative: Schmitt, Armand

(57) **Abstract**

A spectroscopic imaging system (30) for analysing light originating from an object in response to illumination light impinging thereon, the system comprising:
means for receiving the light originating from the object in response to the illumination light and for directing the light originating from the object upon filtering means (32);
the filtering means comprising an acousto-optic tunable filter (AOTF) (32), wherein passing the light originating from the object through the AOTF (32) results in spatially separated diffracted and undiffracted beams (38; 40) exiting the AOTF (32);
a first output optic (42) for producing an image of a diffracted beam (38) exiting the AOTF (32);
a second output optic (46) for producing an image of an undiffracted beam (40) exiting the AOTF (32).

## Description

### FIELD OF THE INVENTION

The present invention generally relates to a spectroscopic imaging system.

### BACKGROUND OF THE INVENTION

Spectroscopic imaging can be used to determine the spatially distributed and chemically distinct species in heterogeneous materials. It is an analytical tool that has been applied at both macroscopic and microscopic levels. At the microscopic level, spectroscopic imaging is widely employed in the medical and biological fields, and includes fluorescence, Raman and Infrared spectroscopy. Indeed, a number of diagnostic techniques are based on optical spectroscopy and have the potential to link the biochemical and morphological properties of tissues to individual patient care.

For example, fluorescence spectroscopy in the ultraviolet (UV) and visible (VIS) spectral regions is widely employed as a diagnostic tool for the detection of pre-cancers and cancers, in vivo.

In vivo fluorescence imaging is typically carried out by means of a spectroscopic imaging system comprising an endoscope, as e.g. described in US 6,212,425. The system comprises an endoscope having a rod-shaped part that can be introduced into a human body, a light guide connection and an eyepiece. In the endoscope, a light guide (e.g. composed of a bundle of glass fibres) guides the light from the light connection to the distal end of the endoscope to be directed towards a to-be-examined tissue area. The endoscope further comprises an objective and an associated image transmitter for guiding the light originating from the tissue area and entering the objective to the proximal end of the endoscope. The image of the tissue area can be recorded with a video camera attached to the eyepiece. For imaging fluorescence spectroscopy, the device is further provided with filter systems that are placed in the illumination optical path (for selecting the illuminating light wavelength) and in the observation optical path (for selecting fluorescence wavelengths). These filters can be removed from the illumination, respectively observation, optical path so as to allow normal observation of the tissue area without leading to colour distortion.

In a typical endoscopic procedure, imaging under "white light" (i.e. broad band light without filters) is first performed to navigate in the body and localise the tissue to be examined. Once the physician has localised the tissue area to be examined, he switches to fluorescence imaging, by setting the filters in place so as to have a substantially monochromatic light illuminating the tissue area and have a short pass filter in the observation beam to view selected fluorescence wavelengths.

A disadvantage of such a system is that in order to switch between normal and fluorescence imaging, the operator has to move the filters. In order to avoid moving the filter out of the observation optical path, a beam splitter can be placed in the observation optical path, so that one part of the emitted light is directed to a video camera and the other part of the emitted light is directed, through the observation filter, to a charge coupled device to capture the spectral image. This however strongly reduces the emitted light intensity and is thus not desired for some applications such as e.g. low-level fluorescence imaging.

Another disadvantageous aspect of the system of US 6,212,425 is low spectral selectivity, which can be remedied by using a filter wheel comprising a number of filters that can be placed in the observation optical path to detect other fluorescence wavelengths. Nevertheless, even if the use of a filter wheel would improve the spectral selectivity of such a system, the operator would still have to make sure that none of the filters is in the observation beam path to obtain a standard (colour) image of the observed tissue.

These problems are similarly encountered with other spectroscopic imaging systems, which employ fixed filters or filter wheels, and wherein a normal image of the to-be-examined object is required or useful to the operator before the spectroscopic analysis.

### OBJECT OF THE INVENTION

An object of the present invention is to provide an improved spectroscopic imaging system that does not exhibit the above-mentioned problems. This object is achieved by a spectroscopic imaging system as claimed in claim 1.

### SUMMARY OF THE INVENTION

According to the present invention, a spectroscopic imaging system comprises means for receiving light originating from an object in response to illumination light impinging thereon and for directing the light originating from the object upon filtering means. The filtering means comprises an acousto-optic tunable filter (AOTF), so that said light originating from the object and passing through the AOTF results in spatially separated diffracted and undiffracted beams exiting the AOTF. The system further comprises a first output optic for producing an image of a diffracted beam exiting the AOTF and a second output optic for producing an image of an undiffracted beam exiting the AOTF.

It will be noted that the diffracted and undiffracted beams are formed by a group of light rays that can be focused in a plane to produce an image of the examined object.

The use of an AOTF in the present system proves extremely advantageous. Indeed, an AOTF allows fast continuous or random-access tuning with high filtering efficiency (normally between 80% and 98%). Since filtering is achieved through a diffraction process, the filtered wavefront can be completely separated from unfiltered light, resulting in zero leakage or cross-talk. This greatly improves the overall signal-to-noise ratio of the system. The AOTF can also support single or simultaneous multiple bandpass filtering, selected sequentially (scanning mode) or directly (random access mode). The AOTF can be amplitude modulated to control its efficiency (gain) over the tuning range, allowing continuous adjustment of dynamic range. It is a solid-state device (no moving parts) that can be easily controlled by a digital or computer interface.

It will further be appreciated that the present system takes advantage of the spatial separation of the diffracted and undiffracted beams exiting the AOTF. This configuration allows to obtain a filtered beam (substantially monochromatic) used for spectral analysis as well as an unfiltered image of the analysed object via the undiffracted beam. This is achieved without use of a beam splitter nor movement of any mechanical part in the system (such as a filter or filter wheel).

As a result, the spectroscopic imaging system according to the present invention permits a precise, fast and highly selective filtering of wavelengths originating from an object to be analysed, and also permits a standard (i.e. non-filtered) observation of the object. The standard observation of the object can be carried out without any beam splitter and without user intervention on interchangeable filters as in conventional systems.

The system of the invention may find application in all fields of spectroscopy where simultaneous or sequential observation of a natural image and a spectral (i.e. filtered) image is desired. Accordingly, the illumination beam may be in the ultraviolet, visible, near-infrared or infrared range. This allows implementation of fluorescence spectroscopy as well as Raman or Infrared spectroscopy. The system is particularly well adapted for fluorescence spectroscopy, in which case the illumination light is substantially monochromatic light, namely in the violet or ultra-violet light range. Indeed, the use of an AOTF greatly improves the overall signal-to-noise ratio of the system, which is essential for low-level fluorescence applications.

The present system can be designed as a modular system, in which case the system preferably comprises an input optic that is adapted to be operatively coupled to other imaging instruments. For example, the present system may be operatively coupled to a standard endoscope, in such a way that the light originating from the illuminated object is received by the endoscope and transmitted to the input optic of the present system.

The system may however alternatively comprise its own means for directing the illumination light towards the object to be analysed. The system may then also comprise its own light source for producing illumination light in the appropriate range, e.g. ultraviolet, visible, near-infrared or infrared.

For observation of the natural image of the object, the light source is preferably also capable of producing white light, alternatively or simultaneously with the illumination light.

A variety of AOTFs can be used in the present system. However, the AOTF preferably is of the non-collinear type and comprises a birefringent crystal and a piezoelectric transducer bonded to one face of the birefringent crystal. A radio frequency (RF) signal source allows applying an RF signal to the birefringent crystal via the transducer. RF control means is provided for controlling the RF signal source and generating an RF signal of desired frequency.

The present system is further advantageously designed to compensate for light dispersion due to travelling through the AOTF. Therefore, the birefringent crystal preferably has a wedge-shape, which is optimised for compensating for colour-shift of light diffracted by the AOTF. The term "colour-shift" conventionally designates the variation in exit angle of the diffracted beams due to the variation of wavelengths. The crystal may be designed to correct the colour-shift of either one of the first-order beams. Dispersion of undiffracted beams is preferably corrected by means of a prism.

Preferably, the system includes an input optic on the input side of the AOTF for providing to the AOTF a substantially collimated beam (wave front) of light originating from the examined object.

The first output optic is preferably arranged to produce an image of either an ordinary or an extraordinary polarised beam exiting the AOTF. This provides a spectral image of the analysed object, which is preferably captured by means of a detector. A charge coupled device (CCD) is preferably used as detector, more preferably an intensified CCD.

The system also preferably comprises a video camera coupled to the second output optic. This allows viewing a standard image of the object.

The second output optic preferably includes a polarizer for blocking undiffracted beams having a predetermined polarization.

The detector (e.g. intensified CCD) and/or the video camera and/or the light source as well as the AOTF are advantageously controlled by a computerized control device that is programmed for switching between navigation any spectral analysis modes, acquiring spectral images, and processing and storing the spectral images. Processing of images can be undertaken to enhance the results of the fluorescence investigation such as the subtraction of the background noise, normalisation of fluorescence maps, and other standard processing. In addition, the resulting processed fluorescence image can be displayed separately or superimposed on the real image (i.e. standard colour or black and white image obtained with the video camera).

### BRIEF DESCRIPTION OF THE DRAWINGS

The present invention will now be described, by way of example, with reference to the accompanying drawings, in which:
FIG.1: is a sketch illustrating the operating principle of an AOTF;
FIG.2: is a sketch illustrating a preferred embodiment of a system according to the invention; and
FIG.3: is a sketch illustrating a system adapted for performing endoscopic fluorescence spectroscopy.

### DETAILED DESCRIPTION OF A PREFERRED EMBODIMENT

Before describing in more detail a preferred embodiment of the present spectroscopic imaging system, the operating principle of an AOTF will first be briefly presented.

### 1. AOTF operating principle

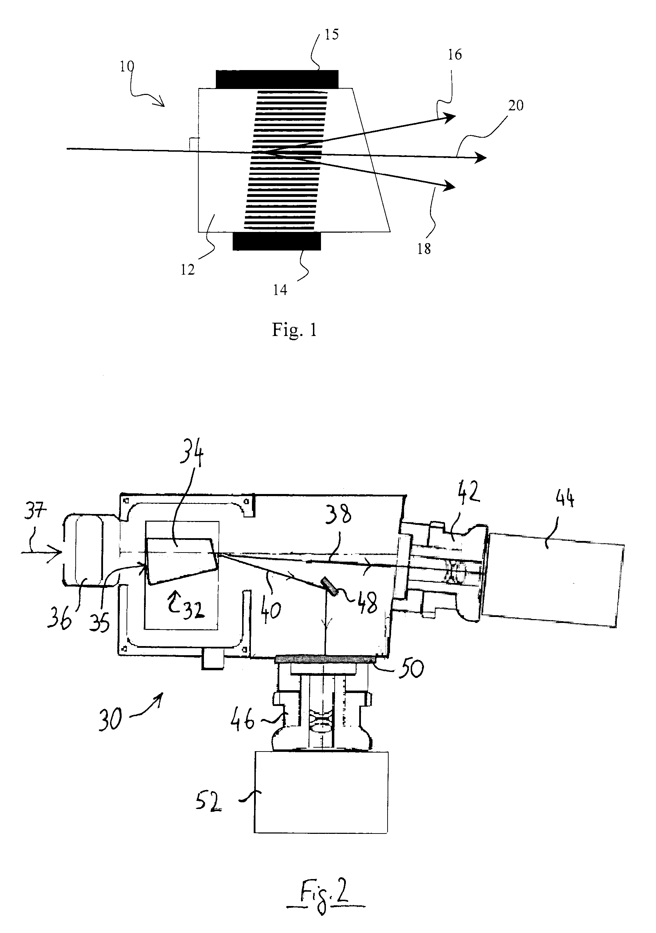

An AOTF is a solid-state electronically tunable spectral bandpass filter, which operates on the principle of acousto-optic interaction in an anisotropic medium. Its operating principle will be explained with regard to Fig.1.

A typical AOTF 10 consists of a birefringent crystal 12 with an acoustic transducer 14 bonded at one face. An acoustic absorber 15 is bonded on the face of the crystal opposed to the transducer 14. When a radio frequency (RF) signal is applied, the transducer 14 generates acoustic waves that propagate in the crystal 12. These propagating acoustic waves produce a periodic modulation of the index of refraction and diffract portions of an incident light beam. For a fixed acoustic frequency, only a narrow band of optical wavelengths are diffracted. As the RF changes, the centre of optical bandpass changes accordingly. This creates a tuneable bandpass filter.

The light diffraction taking place within the AOTF is determined by the momentum matching between the light and the acoustic wave vectors. A specific wavelength can be effectively diffracted by the AOTF with respect to a corresponding driving RF signal. If unpolarised light is passed through the AOTF, then output light exiting the AOTF consists of spatially separated diffracted and undiffracted light, respectively forming the first-order and zero-order. More precisely, the beams exiting the AOTF are:
- a filtered, diffracted monochromatic beam that is o-ray polarised, indicated 16 in Fig.1;
- a filtered diffracted monochromatic beam that is e-ray polarised, indicated 18 in Fig.1;
- two undiffracted (unfiltered) light beams that are orthogonally polarisedand they can be considered to contain the total sum of the unfiltered, undiffracted light energy. These two light beams are represented in Fig.1 by a single beam 20.

As can be seen, the three output beams 16, 18 and 20 are spatially separated, since they exit the crystal 12 at different angles.

It is to be noted that the centre wavelength of the optical pass-band of the AOTF is inversely proportional to the applied radio frequency and can be changed electronically with very fast response time. The AOTF can be operated in sequential or random wavelength access and multi-wavelength modes. The device can also be amplitude modulated, thus controlling its filtering efficiency and bandwidth over the tuning range, and due to its electronic input, it is relatively easy to interface to a computer. This is of great advantage when real time processing is required, for example automatic calibration by normal i-sation of the filtering efficiency over the tuning range.

### 2. Description of a preferred system

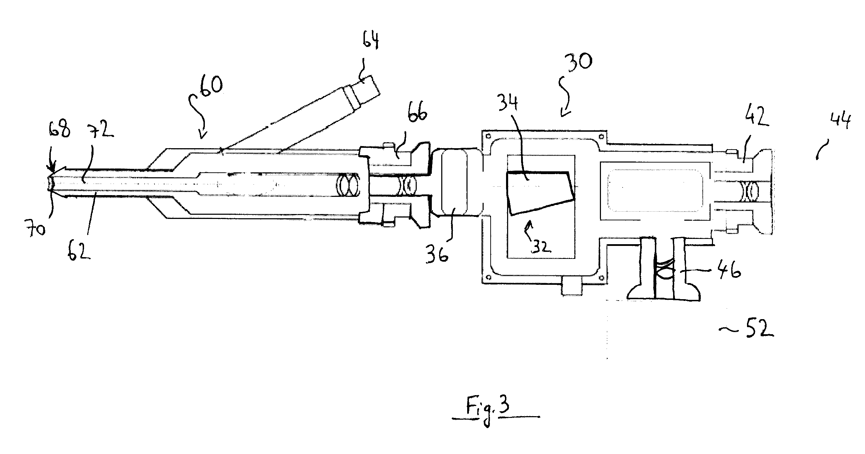

Fig.2 schematically shows a preferred embodiment 30 of a spectroscopic imaging system in accordance with the invention. Reference sign 32 generally indicates an AOTF (not shown in detail) comprising a birefringent crystal 34 (e.g. TeO₂) and a piezoelectric transducer bonded to one side of the crystal 34 in such a way as to preferably form a non-collinear AOTF. An absorber is preferably bonded on the face of the crystal 34 opposed to the transducer 14. A RF signal source allows applying a RF signal to the birefringent crystal 34 via the transducer. The AOTF 32 also comprises RF control means for controlling the RF signal source and generating a RF signal of desired frequency.

An input optic 36 placed before the AOTF 32 receives light originating from the object to be analysed (indicated by arrow 37) and redirects it to the crystal 34 input side 35. The input optic 36 preferably has a telescope configuration composed of e.g. two or more lenses and provides substantially coll i-mated light to the crystal 34 in order to maximise diffraction efficiency and minimise blur in the image. In addition, the input optic 36 allows the adjustment of the diameter of the object light in accordance with the active aperture of the AOTF crystal 34.

As the light originating from the object is passed through the AOTF 32, spatially separated diffracted and undiffracted beams exit the crystal 34. In Fig.2, reference sign 38 indicates the ordinary-polarised diffracted beam, which contains wavelengths that have been filtered by the AOTF 32. This beam 38 thus contains a spectral image of the object under analysis. The undiffracted (unfiltered) beam is designated 40, and contains non-filtered wavelengths, which when imaged will provide a standard (by opposition to "filtered") image of the object. As already explained, the undiffracted beam 40 is generally comprised of two undiffracted beams that are orthogonally polarised, but that exit the crystal 34 at approximately the same angle. Although not represented in Fig.2, a diffracted extraordinary-polarised beam also exits the AOTF, at an angle different from that of the two other beams 38 and 40; this beam is not used in the present embodiment.

It will be understood that the AOTF only outputs the diffracted beams when it is active, i.e. when an RF signal is applied to the crystal 34. In such a case, the diffracted beams are comprised of the filtered wavelengths and the undiffracted beams 40 are comprised of all the remaining wavelengths. If no RF signal is applied to the crystal 34, then there is no diffracted beams and only the zero-order beams 40 exit the AOTF, which are comprised of all the wavelengths.

The present system 30 further comprises a first output optic associated with the O-polarised diffracted beam 38 to produce an image thereof. A second output optic is associated with the undiffracted beam 40 to produce an image thereof. The system 30 thus has a two output channel configuration, that allows to obtain a spectral image of the object (via the first output optic) and a standard image of the of the object (via the second output optic), without requiring a beam splitter or user intervention on moveable filters. In this connection, it will be understood that, if the AOTF 32 is inactive, then all wavelength entering the AOTF 32 will exit as undiffracted beams. This allows standard observation (with normal colours) of an object under e.g. white light illumination. Now, in the case of fluorescence imaging where the object under analysis is illuminated with both white light and UV-light, the AOTF 32 is activated and first- and zero-order beams exit the AOTF 32. In such a case, the spectral image is obtained by one of the selected diffracted beam 38 that contain the O-polarized filtered wavelengths. While performing the spectral analysis, it is simultaneously possible for the operator to observe a standard image of the object via the undiffracted beams. This standard image will of course lack the filtered wavelengths (as explained above). However the missing wavelength range is generally so narrow, that it is in fact barely noticeable by the naked eye and therefore does not hinder standard image observation.

In the present embodiment, the first output optic simply consists of an eyepiece 42 that is aligned with the O-polarised diffracted beam 38 exiting the AOTF 32. An intensified charge coupled device 44 is coupled to the eyepiece 42 by means of an adapter. This permits capturing the spectral images and process them in a control system.

The second output optic also includes an eyepiece 46, which is placed at a distance from the first eyepiece 44 for ease of access. The second output optic also includes a mirror 48 that is positioned in such a way that the undiffracted beam 40 exiting the AOTF 32 impinges thereon and is redirected towards the eyepiece 46. Although not represented, the second output optic further includes a prism or double prism to compensate the dispersion of the undiffracted beam caused by the AOTF 32. Alternatively, a prism could do the job alone, i.e. both redirect the beam towards the eyepiece 44 and compensate the colour dispersion. To improve the image quality, the second output optics preferably further comprises a polarizer 50, e.g. arranged before the eyepiece 46, to block one of the two undiffracted beams. A video camera 52 is attached to the eyepiece 46 to allow viewing of the standard colour images on a monitor.

It will be noted that the crystal 34 preferably has a wedge shape, which is designed to compensate for the colour-shift of the O-polarised diffracted beam 38.

As illustrated in Fig.2, the AOTF 32, input and output optics are advantageously integrated in a housing, so as to provide a modular spectroscopic device that can be coupled to a variety of spectroscopic instruments to be used in the different fields of spectroscopy.

### 3. Application to fluorescence spectroscopy

One of the more fascinating applications of imaging spectroscopy is real-time fluorescence-based systems used e.g. as a diagnosis tool for identifying cancers and precancers, or during cancer surgery to delineate remaining traces of malignant cells. This typically involves illuminating the biological tissue with light of an appropriate wavelength (normally in the UV or blue-green range of the spectrum) and observing the resulting fluorescence. Tissue fluorescence occurs at longer wavelengths than the excitation ill umination and is typically much weaker, so that spectroscopic techniques are required for its detection.

In this particular field of cancer diagnosis, fluorescence-based methods can be divided in two groups. The first group of methods observes fluorescence from medications administered to the patient which have accumulated in tumor tissues. The second group of methods observes endogenous fluorescence or autofluorescence arising from substances natural to the tissue itself which change their relative concentrations when the tissue becomes e.g. dysplastic. Indeed, the fluorescence signature of benign or malignant cells have been shown to sufficiently differ to allow discrimination. Today, fluorescence spectroscopy is often performed in vivo with fluorescence imagining endoscopes which produce detailed, two dimension images.

Fig.3 illustrates a preferred embodiment of the present spectroscopic imaging system adapted for endoscopic imaging fluorescence spectroscopy. A standard endoscope, e.g. a laparoscope, is indicated 60 in Fig.2. Such an endoscope typically comprises a rod-shaped part 62 that can be introduced into a human body, a light guide connection 64 and an eyepiece 66. In the endoscope 60, a light guide (not shown) guides the light from the light guide connection 64 to the distal end 68 to be directed towards a to-be-examined tissue area. The light guide may consists of two bundles of glass fibres to guide both white and UV light into the body. Other instruments may include a biopsy channel that can also be used in conjunction with optical fibres to guide UV and/or white light. The endoscope further comprises an objective 70 and an associated image transmitter 72, to guide the light originating from the tissue area and entering the objective 70, to the proximal end of the endoscope 60. A light source (not shown) is connected to the light guide connection 64 to provide the desired light to the distal end 68 of the endoscope 60. The light source may comprise an excitation light source such as e.g. a laser or a filtered lamp to produce an illumination light in the appropriate spectral range, typically in the UV or blue-green range. The light source preferably also comprises a lamp for producing a white light (broadband) to permit navigation in the body under normal illumination.

Reference sign 30 indicates the spectroscopic system described hereabove (section 2). As can be seen, the input optic 36 of the system 30 is coupled to the eyepiece 66 of the endoscope 60, so that the images of the observed object are passed through the AOTF 32. As already explained, an intensified CCD 44 is attached to the eyepiece 42 of the first output optic and a video camera 52 is attached to the eyepiece 46 of the second output optic. This system thus permits to observe spectral and coloured images of an object to be examined, without having to move any mechanical part in the system (such as a filter or filter wheel) or using a beam splitter.

The intensified CCD 44, the video camera 52, the light source and the AOTF are advantageously controlled by a computerized control device that is programmed for switching between navigation any spectral analysis modes, acquiring spectral images, and processing and storing the spectral images. Processing of images can be undertaken to enhance the results of the fluorescence investigation such as the subtraction of the background noise, normalisation of fluorescence maps, and other standard processing. In addition, the resulting processed fluorescence image can be displayed separately or superimposed on the real image (i.e. standard colour or black and white image obtained with the video camera).

In practice, an operator first actuates the white light as in standard endoscopy, no RF signal is applied to the AOTF 32. Only the undiffracted (zero-order) beams exit the crystal 34. A non filtered image is thus captured by the video camera 52 and displayed on a monitor for real-time imaging. This permits the examiner to navigate in the patient's body and locate the tissue to be examined.

Once the area to be examined is identified, a fluorescence analysis can be carried out. The operator activates the RF source connected to the AOTF and the UV source to excite the tissue under examination. A fluorescence signal is emitted and passes through the AOTF. Two diffracted beams exit the crystal with different deflection angles. As mentioned, only the filtered beam 38 that is O-polarised is taken into account and imaged by the intensified CCD 44.

This sequential operation mode, where the operator switches between white light illumination and UV excitation, is necessary when low-level fluorescence signals are to be measured.

An alternative mode of operation is possible, however, where both light sources are switched on together, allowing simultaneous white light illumination and UV excitation and therefore simultaneous colour image and fluorescence image acquisition, with no change in the device configuration. This operation mode can be applied when the fluorescence signal is sufficiently high so that the filtered white light doesn't degrade the signal-to-noise ratio.

The AOTF can also support single or (simultaneous) multiple bandpass filtering, selected sequentially (scanning mode) or directly (random access mode). The multiple bandpass-filtering mode is useful if the fluorescence spectrum shows more than one wavelength of interest. Suppose that a chromophore emits fluorescence with peaks at two different wavelength bands. It will be practical to take into account both bands. Generally, in a standard fluorescence instrument, a first signal is acquired by using a filter centred at the first wavelength and the image stored, then the filter is changed and second image is acquired at the second wavelength. Once the two images acquired, they are combined to take into account the two fluorescence bands in the spectra. With the present system, the two wavelengths can be filtered simultaneously and the resulting filtered signal contains information on all the wavelengths of interest. This is a considerable advantage with regard to operation time and costs.

## Claims

1. A spectroscopic imaging system for analysing light originating from an object in response to illumination light impinging thereon, said system comprising:
means for receiving said light originating from said object in response to said illumination light and for directing said light originating from said object upon filtering means;
said filtering means comprising an acousto-optic tunable filter (AOTF), wherein passing said light originating from said object through said AOTF results in spatially separated diffracted and undiffracted beams exiting said AOTF;
a first output optic for producing an image of a diffracted beam exiting said AOTF;
a second output optic for producing an image of an undiffracted beam exiting said AOTF.

2. The system according to claim 1, comprising means for directing illumination light towards said object to be analysed.

3. The system according to claim 1 or 2, wherein said illumination light is in the ultraviolet, visible, near-infrared or infrared range.

4. The system according to claim 1, 2 or 3, wherein said illumination light is substantially monochromatic light, preferably in the violet or ultra-violet range.

5. The system according to any one of the preceding claims, comprising a light source for producing said illumination light.

6. The system according to claim 5, wherein said light source further produces white light, either alternatively or simultaneously with said illumination light.

7. The system according to any one of the preceding claims, wherein said AOTF comprises:
a birefringent crystal;
a transducer bonded to one face of said birefringent crystal;
a radio frequency signal source for applying a radio frequency signal to said birefringent crystal via said transducer;
radio frequency control means for controlling said radio frequency signal source and generating a radio frequency signal of desired frequency.

8. The system according to claim 7, wherein said birefrigent crystal has a wedge shape designed for compensating for the colour-shift of light diffracted by said AOTF.

9. The system according to any one of the preceding claims, comprising means for compensating for dispersion of light passed through said AOTF.

10. The system according to any one of the preceding claims, comprising an input optic before said AOTF for providing to said AOTF a substantially collimated beam of said light originating from said object.

11. The system according to any one of the preceding claims, wherein said first output optic is arranged to produce an image of either an ordinary or extraordinary polarised beam exiting said AOTF.

12. The system according to any one of the preceding claims, comprising a detector for capturing an image produced by said first output optic.

13. The system according to claim 12, wherein said detector comprises a charge coupled device, more preferably an intensified charge coupled device.

14. The system according to any one of the preceding claims comprising a video camera for viewing an image produced by said second output optic.

15. The system according to any one of the preceding claims, wherein said second output optic include a polarizer for producing an image based on undiffracted beams exiting the AOTF having a predetermined polarity.

16. The system according to any one of the preceding claims, wherein said second output optic comprises a prism to redirect and/or correct the dispersion of said undiffracted beams exiting said AOTF.

17. The system according to any one of the preceding claims, comprising an endoscope,
wherein said means for directing said illumination light towards said object to be analysed and said means for receiving said light originating from said object in response to said illumination are integrated in said endoscope; and
said filtering means and first and second output optic are integrated in a module that is operatively connected to said endoscope.

18. The system according to any one of the preceding claims, wherein said first and/or second output optic include an eyepiece.
